# EUROPEAN PATENT APPLICATION

(11) **EP 2 140 874 A1**
(43) Date of publication of application: **06.01.2010**
(21) Application number: 08740701.1
(22) Date of filing: 21.04.2008
(51) Int. Cl.: A61K 38/00, A61P 1/16

(54) **REMEDY FOR ACUTE HEPATITIS OR PREVENTIVE/REMEDY FOR FULMINANT HEPATITIS**

(30) Priority: 23.04.2007 JP 2007112690
(71) Applicant: Nihon Pharmaceutical Co., Ltd., Tokyo 101-0031 (JP)
(72) Inventor: SASAKI, Tetsuya, Narita-shi Chiba 286-0825 (JP); HARA, Naoko, Narita-shi Chiba 286-0825 (JP); ISHIKAWA, Makoto, Izumisano-shi Osaka 598-0061 (JP)
(74) Representative: Escher, Thomas
(86) International application number: PCT/JP2008/057656
(87) International publication number: WO 2008/133225

(57) **Abstract**

When acute hepatitis progresses to fulminant hepatitis, a large amount of hepatic cells are rapidly broken, and as a result, the prognosis is seriously worsened. Thus, it is important to prognose the progress of acute hepatitis into fulminant hepatitis at an early stage and quickly start an appropriate treatment therefor. Although the prognosis of progress into fulminant hepatitis becomes possible owing to recent advances in test methods and diagnostic techniques, there has been no appropriate prophylactic/therapeutic agent for fulminant hepatitis. The present invention provides a therapeutic agent for acute hepatitis or a prophylactic/therapeutic agent for fulminant hepatitis with little side effect. The problem of the invention was solved by using a composition containing apolipoprotein A-II.

SEQUENCE LISTING

<110> Nihon Pharmaceutical Co., Ltd.
<120> Therapeutic product for acute hepatitis or prophylactic and therapeutic product
for fulminant hepatic failure

<160> 4
<210> 1
<211> 22
<212> DNA
<213> Artificial Sequence
<400> 1
caggcaaagg agccatgtgt gg   22

<110> Nihon Pharmaceutical Co., Ltd.
<120> Therapeutic product for acute hepatitis  or prophylactic and therapeutic
product
for fulminant hepatic failure

<160> 4
<210> 2
<211> 20
<212> DNA
<213> Artificial Sequence
<400> 2
tcactgggtg gcaggctgtg   20

<110> Nihon Pharmaceutical Co., Ltd.
<120> Therapeutic product for acute hepatitis  or prophylactic and therapeutic
product
for fulminant hepatic failure

<160> 4
<210> 3
<211> 77
<212> PRT
<213> Homo sapiens
<400> 3
Gln Ala Lys Glu Pro Cys Val Glu Ser Leu Val Ser Gln Tyr Phe Gln
1               5                   10                  15
Thr Val Thr Asp Tyr Gly Lys Asp Leu Met Glu Lys Val Lys Ser Pro
            20                  25                  30
Glu Leu Gln Ala Glu Ala Lys Ser Tyr Phe Glu Lys Ser Lys Glu Gln
        35                  40                  45
Leu Thr Pro Leu Ile Lys Lys Ala Gly Thr Glu Leu Val Asn Phe Leu
    50                  55                  60
Ser Tyr Phe Val Glu Leu Gly Thr Gln Pro Ala Thr Gln
 65                  70                  75      77

<110> Nihon Pharmaceutical Co.,Ltd.

<120> Therapeutic product for acute hepatitis  or prophylactic and therapeutic
product
for fulminant hepatic failure

<160> 4
<210> 4
<211> 255
<212> DNA
<213> Artificial Sequence
<400> 4
atgcaagcca aggagccctg cgtcgagtcc cttgtctccc aatacttcca aaccgtcacc    60
gactacggta aggaccttat ggagaaggtc aagtcccccg agcttcaagc cgaggccaag   120
tcctacttcg agaagtccaa ggagcaactt acccccctta tcaagaaggc cggtactgag   180
cttgtcaact tcctttccta cttcgtcgag cttggtactc aacccgccac ccaacaccac   240
caccaccacc actga                                                    255

## Description

### Technical Field

The present invention relates to a therapeutic agent for acute hepatitis or a prophylactic/therapeutic agent for fulminant hepatitis, which contains apolipoprotein A-II as an active ingredient.

### Background Art

The liver plays an important role for life activities, for example, synthesis of important substances for the body and excretion of waste products. Hepatitis is a liver dysfunction caused by hepatic cells damaged by viruses, alcohol, drugs and the other like. The symptoms of hepatitis are malaise, pophagia, nausea, fever, jaundice and so on. Hepatitis that has developed within 6 months is particularly called acute hepatitis. Hepatitis is called fulminant hepatitis showing a prothrombin time of 40% or less, which has developed encephalopathy of II-grade or more hepatic coma based on high-degree hepatocellular dysfunction within 8 weeks after onset of symptoms of acute hepatitis.
In fulminant hepatitis, a large amount of hepatic cells are damaged rapidly, thus resulting in failure to keep up with the growth and regeneration of the cells, and very poor prognosis is brought about. Accordingly, early prognosis of progress of acute to fulminant hepatitis and prompt suitable treatment therefor are important.

The number of patients with acute hepatitis in Japan reaches 350,000 annually, among which the number of patients progressing to fulminant hepatitis is said to be several thousands annually.
It is said that together with the immune response to a body, various factors in local areas of the liver are closely associated with the occurrence mechanism of fulminant hepatitis, but there still remain unclear points.

For prognosis of progress of acute hepatitis into fulminant hepatitis, attention should be paid to subjective symptoms of the patient and to various abnormal values in liver function tests.
Generally, the progress of acute hepatitis into fulminant hepatitis proceeds following the deterioration, in hepatic synthetic function, caused by sudden breakage of hepatic cells and subsequent deterioration in hepatic detoxification and metabolic function. Sudden breakage of a large amount of hepatic cells appears as subjective symptoms such as strong malaise and hypophagia, and jaundice develops as levels of aminotransferase such as alanine aminotransferase (ALT) and aspartate aminotransferase (AST) and a level of LDH (lactase dehydrogenase) increase. Neurological symptoms of I- or II-grade hepatic coma appear due to deterioration in hepatic detoxification and metabolic function, and thus electroencephalographic examination is also important in diagnosis. Recently, diagnostic kits and diagnostic markers which prognose and diagnose clinical conditions of hepatitis such as fulminant hepatitis have also been reported (Patent Document 1). From the indication of these symptoms, examination results, and improvement of diagnostic techniques, the prognosis of progress of acute hepatitis into fulminant hepatitis becomes possible with very high probability.

Treatment of acute hepatitis is based on resting in bed, but when clinical conditions are progressing, symptomatic treatment using a liver protection medicine such as a glycyrrhizin preparation is conducted. As a therapeutic agent for acute hepatitis or fulminant hepatitis, a pharmaceutical preparation containing at least one selected from alanine, glutathione and ornithine (Patent Document 2), a pharmaceutical preparation containing a platelet activating factor antagonist (Patent Document 3) and a pharmaceutical preparation containing an insulin-like growth factor or its derivative (Patent Document 4) have been proposed, but are not practically used because their effect is not sufficient.
As therapies for fulminant hepatitis, symptomatic treatments such as plasma exchange, blood transfusion, hemodialysis, and the like have been performed. Recently, hepatic transplantation has also been attempted, but poor prognosis results and the degree of survival is said to be about 30%. When fulminant hepatitis is caused by a virus, interferon therapy has been attempted as antiviral therapy, but produces side effects such as fever, arthritis, myalgia, decreases of leukocytes and platelets, loss of appetite, weight loss, loss of hair, thyroid dysfunction, myocardial damage, worsening of diabetes, and proteinuria, and moreover, its effect cannot be said to be satisfactory.
Patent Document 1: JP-A 2008-17777
Patent Document 2: JP-A 5-221858
Patent Document 3: JP-A 7-304689
Patent Document 4: JP-A 9-143094

### Disclosure of Invention

### Problems to be Solved by the Invention

Under these circumstances, there has been an eager desire for development of a therapeutic agent for acute hepatitis or a prophylactic/therapeutic agent for fulminant hepatitis with little side effect, which prognoses the progress of acute hepatitis into fulminant hepatitis and quickly starts treatment thereby preventing hepatitis from becoming fulminant, and has a therapeutic effect on hepatitis even after becoming fulminant.

### Means for Solving the Problem

The present inventors have made extensive study for the action, on the living body, of apolipoprotein A-II as a component of high-density lipoprotein (HDL) in blood, and as a result, unexpectedly have found that apolipoprotein A-II is effective in treating acute hepatitis and in preventing and treating fulminant hepatitis without producing side effects. On the basis of this finding, the present inventors have made further study to complete the present invention.
That is, the present invention relates to a therapeutic agent for acute hepatitis or a prophylactic/therapeutic agent for fulminant hepatitis, which comprises apolipoprotein A-II as an active ingredient.

Apolipoprotein A-II that is the active ingredient of the present invention is a major constituent of high-density lipoprotein (HDL).
About 50% of the chemical composition of HDL is a protein, and the remainder (about 50% by weight) is a lipid component based on phospholipid and cholesterol ester. In the protein component of HDL, apolipoprotein A-II is contained in the largest amount next to apolipoprotein A-I and accounts for about 20% by weight of the whole protein component.
The chemical structure of apolipoprotein A-II has already been elucidated, indicating that apolipoprotein A-II is a polypeptide having a molecular weight of about 8700, consisting of 77 amino acids, and usually exists in blood as a dimer structure bound by disulfide bonding via a cysteine residue at position 6 (The Lipid, Vol. 2, No. 3, 243-249, 1991-4).
With respect to the physiological role of apolipoprotein A-II, there are reports that apolipoprotein A-II is substituted for apolipoprotein A-I thereby activating liver lipase or suppressing the activity of LCAT (lecithin-cholesterol acyltransferase) in an in vitro experiment, and also that apolipoprotein A-II inhibits the activation of coagulation factor V by coagulation factor VIIa, but the in vivo role thereof is still not revealed.
It has also be reported that the concentration of apolipoprotein A-II in blood tends to increase in patients with hyperlipidemia, while the concentration of apolipoprotein A-II in blood tends to decrease in patients with hepatic diseases (for example, acute hepatitis, chronic hepatitis, hepatic cirrhosis, and liver cancer) (Clinical Pathology XXIX:2, 135-138 (1981)). However, the fact that apolipoprotein A-II decreases in patients with hepatic diseases was merely described, and it is not described that if patients which may have hepatic diseases, particularly acute hepatitis and fulminant hepatitis, are administered with apolipoprotein A-II, hepatitis can be prevented or treated.

Apolipoprotein A-II as the active ingredient of the present invention can be isolated from human plasma by usual protein separation/purification methods or can be produced by genetic engineering techniques using organisms. For example, JP-A 63-237789 describes that apolipoprotein A-II can be produced by culturing genetically recombinant cells produced by integrating an expression vector encoding a gene of apolipoprotein A-II in prokaryotic cells such as Escherichia coli, Bacillus subtilis and streptomyces carbophilus or in eukaryotic cells such as yeasts.
Even when apolipoprotein A-II is a substance produced in an animal body or in animal cells, an analog derived from apolipoprotein A-II by deleting its partial structure by genetic engineering techniques, or a modified product thereof having its constituent amino acids modified or replaced by other amino acids or an analog having its sugar chain modified or deleted by genetic engineering techniques, the apolipoprotein A-II can be used as long as it has a prophylactic/therapeutic effect on acute hepatitis or fulminant hepatitis. With respect to its purity, apolipoprotein A-II may be one purified to such a degree that a single band is shown for example in reduced gel electrophoresis, and there is no necessity for limitation of its purification method or its starting material.

Typical methods for producing apolipoprotein A-II include a method of separation and purification from human plasma or a derivative thereof (for example, Analytical Biochemistry 178, 301-305 (1989)) and a method of production with genetic engineering techniques. The derivative of human plasma includes, for example, a precipitated fraction IV-1 obtained by cone low-temperature ethanol fractionation of human plasma and precipitated fraction B obtained by a low-temperature ethanol fractionation method using a method of Kistler and Nitschmann.

Specifically, the method for producing apolipoprotein A-II from human plasma or a human plasma fraction derived therefrom includes the following method.
First, human plasma or a human plasma fraction derived containing apolipoprotein A-II therefrom is dissolved in a buffer solution at low temperature, for example at 10°C or less. Then, an ethanol/chloroform solution for example is added to and mixed with the above solution which is then centrifuged to remove a lipid component in a lower layer, thereby recovering a protein component in a supernatant. Ethanol is added to the recovered supernatant which is then centrifuged to remove precipitated high molecular protein, and the supernatant is recovered. Ethanol is added again to the recovered supernatant which is then centrifuged to remove precipitated A-I, and a supernatant containing apolipoprotein A-II is recovered. The supernatant containing apolipoprotein A-II is subjected to ultrafiltration treatment with a sodium chloride solution to remove the ethanol. Thereafter, the sample solution is subjected to ultrafiltration treatment with a phosphate buffer and then concentrated to a suitable concentration. An ethanol/chloroform solution is added to and mixed with the insufficiently delipidated, purified apolipoprotein A-II followed by centrifugation to remove a lipid component in a lower layer. The apolipoprotein A-II in a supernatant is subjected to ultrafiltration in the same manner as above to remove the ethanol, and the sample solution is concentrated to a suitable concentration. By this operation, purified apolipoprotein A-II is obtained.

Another preferable example of the method for producing apolipoprotein A-II is a method using genetic engineering techniques.
For production of apolipoprotein A-II by genetic engineering techniques, cDNA encoding a human apolipoprotein A-II gene is first obtained from a DNA library, and the gene is amplified with suitable primers, thereby obtaining a polynucleotide encoding apolipoprotein A-II. The polynucleotide is cloned by insertion into a cloning vector, and then the polynucleotide encoding apolipoprotein A-II is cut off and inserted into a suitable expression vector by a method known per se. This expression vector is transformed into suitable host cells (for example, prokaryotic cells such as Escherichia coli and Bacillus subtilis or eukaryotic cells such as yeasts, insect cells and mammalian cells), and apolipoprotein A-II can be obtained by isolation and purification from a culture of the transformed cells.
The host cells are preferably bacteria such as Bacillus brevis and yeasts such as Schizosaccharomyces pombe.

When apolipoprotein A-II is produced by microorganisms of the genus Bacillus or the like by genetic engineering techniques, an expression vector having an origin capable of replication in a microorganism, a promoter, a ribosomal binding site, a DNA cloning site, a terminator and the like is used to prepare an expression vector in which the polynucleotide encoding apolipoprotein A-II has been integrated. After a host cell is transformed with this expression vector, the resulting transformant is cultured, and the objective protein can be isolated and purified from its culture, thereby obtaining apolipoprotein A-II. When an arbitrary coding region is added between an initiation codon and a termination codon and expressed, a protein fragment containing the arbitrary region can be obtained. Alternatively, apolipoprotein A-II can be expressed as a fusion protein with another protein. This fusion protein can be cleaved with a suitable protease to give a recombinant apolipoprotein A-II.

A pharmaceutical preparation containing apolipoprotein A-II includes an aqueous solution, a suspension, a lyophilized preparation and the like, and these preparations can be obtained in a usual manner for pharmaceutical manufacturing after mixing apolipoprotein A-II with pharmaceutically acceptable additives (a diluent, a pH regulating agent, a tonicity agent, a surfactant and the like) as necessary. The lyophilized preparation is dissolved just before use in a solution such as distilled water for injection.

When the recombinant apolipoprotein A-II is produced in eukaryotic cells, the polynucleotide encoding apolipoprotein A-II is inserted into a eukaryotic expression vector harboring a promoter, a splicing region, a poly (A) addition site and the like, to prepare a recombinant vector. This vector is introduced into a eukaryotic cell, and the objective protein may be isolated and purified from its culture. The eukaryotic cell includes generally used cells, for example, mammalian cultured cells such as simian renal cell COS7 and Chinese hamster ovary cell CHO, budding yeast, fission yeast, silkworm cells and xenopus oocytes. However, any eukaryotic cells may be used as long as recombinant apolipoprotein A-II can be expressed. For introducing the expression vector into a eukaryotic cell, known methods such as electroporation, a calcium phosphate method, a liposome method and a DEAE-dextran method can be used.

After the recombinant apolipoprotein A-II is expressed in prokaryotic cells or eukaryotic cells, the objective protein can be isolated and purified from the culture by a combination of known separation procedures. Separation techniques include, for example, treatment with a denaturant such as urea or with a surfactant, sonication, enzymatic digestion, salting-out or solvent precipitation, dialysis, centrifugation, ultrafiltration, gel filtration, SDS-PAGE, isoelectric focusing electrophoresis, ion-exchange chromatography, hydrophobic chromatography, affinity chromatography, reverse phase chromatography, and the like.

The protein expressed in the host cell may be subjected after translation to various modifications in the cell. Accordingly, the recombinant apolipoprotein A-II used in the present invention includes those proteins that have been subjected to posttranslational modifications such as elimination of N-terminal methionine, N-terminal acetylation, sugar-chain addition, limited proteolysis with intracellular proteases, myristoylation, isoprenylation, and phosphorylation.

The apolipoprotein A-II obtained in this manner scarcely contains impurities. This apolipoprotein A-II can be formed into a pharmaceutical preparation by mixing it with pharmacologically acceptable excipients. The excipients can be selected appropriately from a broad range of materials, depending on the administration form of the pharmaceutical preparation. The apolipoprotein A-II can be stored if necessary together with suitable stabilizers such as salts, amino acids, organic acids, sugar alcohols, proteins such as albumin, and polyols.

Although the route of administration of the pharmaceutical preparation of the present invention, whether oral or parenteral, is not particularly limited, parenteral administration by intravenous injection, intramuscular injection, subcutaneous injection or intradermal injection is preferable, and particularly intravenous injection is preferable. For preparation of an injection, a predetermined amount of the purified apolipoprotein A-II may be mixed under aseptic conditions with injection distilled water, upon which a buffer agent, a tonicity agent, a stabilizer and the like are preferably incorporated in suitable amounts into it. Specifically, sodium phosphate, sodium citrate, sodium chloride, and L-glutamic acid are preferably used.

The dosage of the therapeutic agent for acute hepatitis or of the prophylactic or therapeutic agent for fulminant hepatitis shall be an amount enough to exhibit the intended effect, but may be varied appropriately depending on a dosage form, an administration method, administration frequency per day, the degree of symptoms, body weight, age, and the like. In the case of intravenous administration into an adult, the preparation is administered in an amount of usually about 1 to 1000 mg/kg, preferably about 3 to 500 mg/kg, in terms of the amount of apolipoprotein A-II. This amount of apolipoprotein A-II can be administered once per day or in two to three divided portions per day.

### Effect of the Invention

The therapeutic agent for acute hepatitis or the prophylactic/therapeutic agent for fulminant hepatitis in the present invention contains apolipoprotein A-II as an active ingredient and can treat acute hepatitis, or prevent or treat fulminant hepatitis safely and effectively with no or little side effect.

### Best Mode for Carrying Out the Invention

Hereinafter, the present invention will be described specifically with reference to the Examples and Experimental Examples.

### Example 1

Preparation of Apolipoprotein A-II-Containing Preparation Human plasma from which hepatitis viruses and other pathogenic microorganisms had been substantially removed was used as a starting material and subjected to Cohn low-temperature ethanol fractionation to give fraction IV-1. 1.2 kg of the fraction IV-1 was dissolved in 2.4 L of a solution regulated at pH 7.8 to 8.2, containing 100 mM tris(hydroxymethyl)aminomethane and 6 M urea, in a cold room at 2 to 8°C. The resulting solution was mixed with an equal volume of an ethanol/chloroform (1/1) solution and then centrifuged at 12000 × g, 4°C, for 10 minutes to recover a protein component in a supernatant. The recovered supernatant, 3.4 L, was compounded with ethanol in a volume of 4.1 L that is 1.2-fold relative to the supernatant, and then centrifuged at 12000 × g, 4°C, for 10 minutes, thereby recovering 6.8 L of a supernatant. Further the recovered supernatant was compounded with ethanol in a volume of 5.4 L that is 0.8-fold relative to the supernatant, and then centrifuged at 12000 × g, 4°C, for 10 minutes, thereby recovering 12 L of an apolipoprotein A-II-containing supernatant. The apolipoprotein A-II-containing supernatant was subjected to ultrafiltration through an ultrafiltration membrane with a cut-off molecular weight of 10,000 (manufactured by Pole) with 120 L of 150 mM sodium chloride solution added into it, thereby removing the ethanol. Then, 20 L of 20 mM phosphate buffer, pH 7, containing 150 mM sodium chloride was added into it, and the sample solution was subjected to ultrafiltration, thereby replacing the medium solution. Thereafter, the sample solution was concentrated by ultrafiltration until the concentration of apolipoprotein A-II reached 4 mg/mL.
Then, 500 ml of the apolipoprotein A-II concentrate thus obtained was mixed with an equal volume of an ethanol/chloroform (1/1) solution and then centrifuged at 12000 × g, 4°C, for 10 minutes, followed by removing a lower layer, to recovery 750 mL of a supernatant. The supernatant was subjected to ultrafiltration through an ultrafiltration membrane with a cut-off molecular weight of 10,000 (manufactured by Millipore) with 25 L of 150 mM sodium chloride solution added into it, thereby removing the ethanol. Then, 20 L of 20 mM phosphate buffer, pH 7, containing 150 mM sodium chloride was added into it, and the sample solution was subjected to ultrafiltration, thereby replacing the medium solution. The sample solution was concentrated by ultrafiltration until the concentrate contained 22 mg/mL of apolipoprotein A-II. The concentrate thus obtained was subjected to aseptic filtration with an aseptic filtration filter having a pore size of 0.22 µm (manufactured by Millipore) to give an apolipoprotein A-II preparation.
Analysis results of the apolipoprotein A-II preparation are shown in Table 1.

**Table 1**

| | |
|---|---|
| Apolipoprotein content (nig/mL) | 21.5 |
| PH | 7.3 |
| Osmotic pressure ratio | 1.0 |
| Endotoxin content (EU/mL) | 0.2 |
| Phospholipid content (mg/mL) | 0.6 |
| Appearance | Transparent liquid with a pale yellow color |
| Ethanol concentration (%) | 0.01 |

The pharmaceutical preparation obtained in this manner can be used directly as an injection.

### Example 2

(1) Construction of an expression vector for recombinant human apolipoprotein A-II The human apolipoprotein A-II gene was cloned by PCR where a human liver cDNA library (product code 9505, manufactured by Takara Bio) was used as a template. In this PCR, a sequence set forth in SEQ ID NO: 1 in the Sequence Listing was used as a forward primer, and a sequence in SEQ ID NO: 2 in the sequence Listing was used as a reverse primer. The resulting PCR fragment was cloned into pCR2.1 vector (manufactured by Invitrogen) by a TA cloning method using a TOPO TA cloning kit (manufactured by Invitrogen).
(2) Preparation of a human apolipoprotein A-II-expressing strain using Bacillus brevis The resulting vector containing the human apolipoprotein A-II gene was used to transform Bacillus brevis to construct an expression strain. The method of constructing the expression strain was carried out in accordance with a method described in Japanese Patent No. 3734593.
   First, a plasmid vector is conducted in accordance with a method described in Example 1 in the patent supra, and then the plasmid vector into which the human apolipoprotein A-II had been inserted was prepared. Then, the plasmid vector was used to transform Bacillus brevis H102 (FERM BP-1087) by electroporation in accordance with a method shown in Example 5 in the patent supra.
(3) This transformant was cultured in a TMN medium at 30°C for 3 days, and then its culture medium was centrifuged to recover a supernatant, thereby obtaining a culture supernatant.
   For confirming the amino acid sequence of the resulting human apolipoprotein A-II, its nucleotide sequence on the pCR2.1 vector was analyzed with model 3100 DNA sequencer (manufactured by ABI) and converted into amino acids, thereby obtaining an amino acid sequence set forth in SEQ ID NO: 3 in the Sequence Listing.
   This amino acid sequence was a sequence identical with the mature protein portion of human apolipoprotein A-II (primary accession number P02652) shown in Swiss-Prot Protein Knowledgebase.

### Example 3

Preparation of an apolipoprotein A-II-containing preparation from a culture supernatant of the recombinant human apolipoprotein A-II-expressing strain (Bacillus brevis)
The Bacillus brevis culture supernatant in which human apolipoprotein A-II had been expressed and secreted was mixed with an equal volume of 20 mM phosphate buffer (pH 6.6 to 7.4) containing 1 M sodium chloride, then stirred, and filtered through a 0.45 µm filter (manufactured by Millipore) thereby removing precipitates. The filtrate was applied onto His trap FF (manufactured by GE Healthcare) equilibrated previously with 20 mM phosphate buffer (pH 6.8 to 7.4) containing 0.5 M sodium chloride. Then, the His trap FF was washed with the same solution as used in equilibration, and further washed with 20 mM phosphate buffer (pH 7.0 to 7.5) containing 0.5 M sodium chloride and 40 mM imidazole. Finally, the His trap FF-adsorbed fraction was eluted with 20 mM phosphate buffer (pH 7.0 to 7.5) containing 0.5 M sodium chloride and 500 mM imidazole. The eluate of the His trap FF-adsorbed fraction was subjected to ultrafiltration through a centrifugal ultrafiltration membrane with a cut-off molecular weight of 10,000 (manufactured by Millipore) with 20 mM phosphate buffer (containing 150 mM sodium chloride) added into it, thereby replacing the solution medium. The sample solution was further concentrated by ultrafiltration until the concentrate contained 13 mg/mL of apolipoprotein A-II. Subsequently, the concentrate was subjected to aseptic filtration with an aseptic filtration filter having a pore size of 0.22 µm (manufactured by Millipore) to give an apolipoprotein A-II preparation.

### Example 4

His trap FF was brought into operation in the same manner as in Example 3, and the composition of the resulting eluate was subjected to ultrafiltration through a centrifugal ultrafiltration membrane with a cut-off molecular weight of 5000 (manufactured by Millipore) with 3.3 mM phosphate buffer (pH 6.8 to 7.4) containing 3 M urea and 0.5 M sodium chloride, thereby replacing the solution medium. This human apolipoprotein A-II-containing fraction was applied onto Macro-prep ceramic hydroxyapatite Type I 40 µm (manufactured by Bio-Rad Laboratories) equilibrated previously with 3.3 mM phosphate buffer (pH 6.8 to 7.4) containing 3 M urea and 0.5 M sodium chloride, and a non-adsorbed fraction was recovered.
Then, the non-adsorbed fraction was subjected to ultrafiltration through a centrifugal ultrafiltration membrane with a cut-off molecular weight of 5000 (manufactured by Millipore) with 20 mM phosphate buffer containing 150 mM sodium chloride, thereby replacing the solution medium. The sample solution was further concentrated by ultrafiltration until the concentrate contained 2 mg/mL of apolipoprotein A-II. Subsequently, the concentrate was subjected to aseptic filtration with an aseptic filtration filter having a pore size of 0.22 µm (manufactured by Millipore) to give an apolipoprotein A-II preparation.

### Example 5

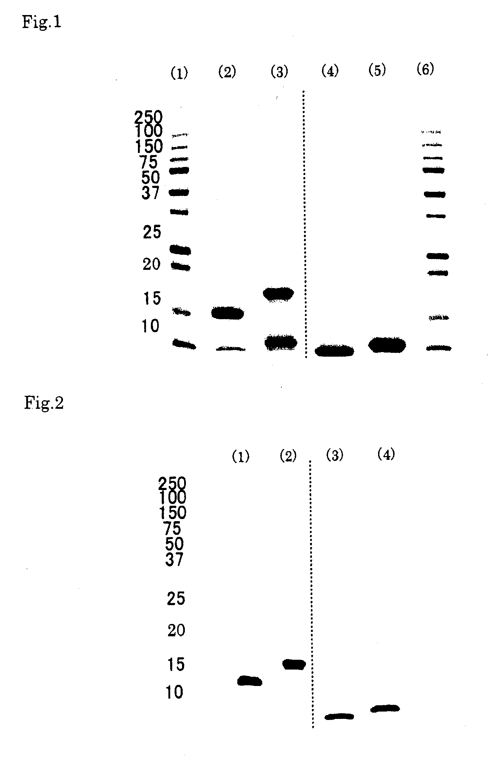

SDS-polyacrylamide gel electrophoresis (SDS-PAGE) The concentrations of the apolipoprotein A-II preparations obtained in Examples 1 and 4 were regulated such that the absorbances of the preparations at OD280 nm became the same. These samples were used as non-reduced samples. Separately, the above preparations were treated with 30 mg/mL DTT (manufactured by Wako Pure Chemical Industries, Ltd.) to give reduced samples. The non-reduced and reduced samples were subjected respectively to SDS-polyacrylamide gel electrophoresis on 15% uniform gel. The results are shown in FIG. 1.

Western blotting The concentrations of the apolipoprotein A-II preparations obtained in Examples 1 and 4 were regulated such that the absorbances of the preparations at OD280 nm became the same. These samples were subjected to SDS-PAGE, and then the protein on each gel was transferred onto a PVDF membrane (manufactured by ATTO) and subjected to primary reaction with a goat anti-human apolipoprotein A-II polyclonal antibody (manufactured by SANTACRUZ BIOTECHNOLOGY). Then, the samples were subjected to secondary reaction with an HRP-labeled rabbit anti-goat immunoglobulin antibody (manufactured by DAKO) and colored by a DAB method. The results are shown in FIG. 2.

As shown in FIGS. 1 and 2, the SDS-PAGE-stained proteins in both the preparations in Examples 1 and 4 reacted with the anti-human apolipoprotein A-II antibody, and the two were thus confirmed to have the same antigenicity. In addition, the two in a reduced form showed a single band in their electrophoretic profile.
When the band positions in the preparations in Examples 1 and 4 in FIGS. 1 and 2 were compared, the band of the preparation in Example 4 was recognized at a position with a slightly larger molecular weight. This is due to addition of the molecular weight of the His-Tag (6 histidine residues) added to the recombinant.

### Example 6

For comparison between the preparations prepared in Examples 1 and 4, a peptide map was prepared to examine them.
Each of the preparations was diluted such that the concentration of apolipoprotein A-II was reduced to 200 µg/mL, and then 4 µL of a sample buffer (350 mM Tris-HCI, pH 6.8, 30% glycerol, 10% SDS, 0.3% BPB, 30% 2-mercaptoethanol) was added to 20 µL of the resulting solution which was then treated at 100°C for 5 minutes, then subjected to SDS-PAGE, and stained with CBB (PhastGel Blue R manufactured by Amersham Biosciences).
The whole amount of a band in the vicinity of 10 kDa on the electrophoresed gel was used in digestion in the gel as follows.
The gel was cut thin to a size of about 1 mm × 1 mm and then washed under stirring (20 minutes × twice) in 1 mL purified water. Then, the gel was washed under stirring (30 minutes) in 50 µL of 0.5 M Tris-HCl buffer (pH 8.5) containing 7 M guanidine and 10 mM EDTA and then washed under stirring (20 minutes × twice) in 1 mL of 50 mM phosphate buffer (pH 7.8) containing 50% acetonitrile and 1 mM EDTA. The gel was further washed under stirring (5 minutes) in 1 mL acetonitrile and then dried with a speed bag. 10 µL of an endoproteinase Glu-C (V8) dissolved in 100 mM phosphate buffer (pH 7.8) containing 2 mM EDTA was added to the dried gel (enzyme : substrate = 1 : 10). The gel was swollen by leaving it on ice for 1 hour and then impregnated with 20 to 60 µL of 100 mM phosphate buffer (pH 7.8) containing 2 mM EDTA, followed by digestion at 25°C for 24 hours. The digested product was concentrated until the total volume was reduced to 50 µL or less.
0.1% trifluoroacetic acid was added to the concentrated sample such that the total volume reached 55 µL, and then 50 µL of the resulting sample solution was analyzed by HPLC under the following conditions.
HPLC conditions
Unit: LC-10 Avp system (manufactured by Shimadzu
Corporation)
Column: 218TP54 (4.6 mm I.D. × 25 cm, manufactured by Vydac)
Column temperature: 40°C
Flow rate: 1 mL/min
Detection wavelength: 215 mm
Mobile phase A: 0.1% trifluoroacetic acid
Mobile phase B: 0.1% trifluoroacetic acid/80% acetonitrile
Gradient conditions: 0/0 → 0/5 → 75/55 → 100/65 → 100/75 →
0/85 → 0/90 (% B/min)

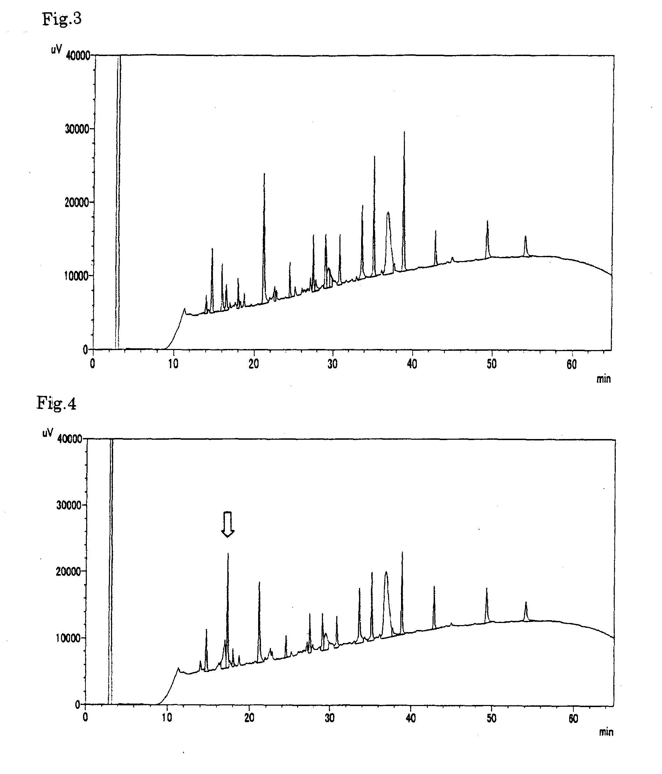

The result in the peptide map in Example 1 is shown in FIG. 3, and the result in the peptide map in Example 4 is shown in FIG. 4. The preparations prepared in Examples 1 and 4 showed the same peptide map except for the His-tag portion, and the preparations in Examples 1 and 4 were thus confirmed to be identical.

### Example 7

Construction of an expression vector for genetically recombinant human apolipoprotein A-II and preparation of an apolipoprotein A-II-expressing strain by using yeast
A human apolipoprotein A-II gene was artificially synthesized. Its nucleotide sequence is as shown in SEQ ID NO: 4 in the Sequence Listing.
The resulting human apolipoprotein A-II gene was inserted into a multicloning site of an expression vector pTI1M5 (Hideki Tohda, "Koso Kogaku News (Enzyme Engineering News)", Vol. 57, April, 2007) to construct an inducible expression vector. This inducible expression vector and a leucine auxotroph complementary vector pAL7 were used to transform Schizosaccharomyces pombe ATCC38399 by a method of Okazaki et al. (Nucleic Acids Res., 18, 6485-6489, 1990) thereby constructing an expression stain. This expression strain was cultured in 5 mL of G418-containing liquid medium YELG10 (0.5% Bacto yeast extract, 3% glucose, 10 µg/mL G418) at 32°C for 7 days. The culture was cultured in 5 mL YPDG100 (1% Bacto yeast extract, 2% Bacto peptone, 2% glucose, 100 µg/mL G418) at 32°C for 7 days and then separated by centrifugation into a bacterial pellet and a supernatant, and the bacterial pellet was recovered. This bacterial sample was suspended in 0.8 mL bacterial disruption buffer (50 mM Tris-HCI, pH 7.5, 0.1 M sodium chloride, a protease inhibitor (manufactured by Roche)), and then the same volume of glass beads were introduced into the microbial suspension which was then disrupted 4 times with a homogenizer, thereby preparing a disrupted bacterial liquid. This disrupted bacterial liquid was centrifuged to recover an apolipoprotein A-II-containing supernatant. The expression level of apolipoprotein A-II was 2 mg/L.

### Example 8

Preparation of an apolipoprotein A-II preparation from cultured bacteria of the genetically recombinant human apolipoprotein A-II-expressing strain (Schizosaccharomyces pombe)
The expression strain prepared in Example 7 was cultured in 300 mL of YPDG100 medium (1% yeast extract, 2% peptone, 2% glucose, 0.1 mg/mL G418). The strain was cultured in 3 mini-jars at 30°C for 66 hours, to give 14.4 g of bacteria (wet weight) in total. The bacteria recovered from each of the mini-jars were suspended in about 16 to 17 mL disruption buffer (50 mM Tris-HCI, pH 7.5, 0.1 M sodium chloride, a protease inhibitor (manufactured by Roche)) during which an additional disruption buffer was gradually added the cell suspension to finally prepare 20 mL of bacterial suspension. To 20 mL of each bacterial suspension thus prepared was added the same volume of glass beads (diameter 0.5 mm), and the suspension was disrupted 4 times with a homogenizer (disrupted at 2500 rpm for 60 seconds) to recover 13 mL of disrupted bacterial liquid. Remaining disrupted bacteria adhered to the glass beads were washed away with 5 mL of an additional disruption buffer, and the wash was combined with the above disrupted bacterial liquid. The bacterial liquid was centrifuged at 5000 g for 10 minutes, to recover a supernatant. The expression level of apolipoprotein A-II was 2 mg/L.
The prepared supernatant was purified with 2 kinds of His-Tag columns as follows. First, 54 mL of the supernatant was applied onto 20 mL TALON Single Step Column (manufactured by Clontech) and eluted with 2 mL elution buffer containing 500 mM imidazole. The eluate was diluted 3-fold with distilled water (DW), then applied onto 0.6 mL His Spin Trap Column (manufactured by GE Healthcare) and eluted with 0.2 mL of elution buffer containing 500 mM imidazole.
Then, the eluate was concentrated while its medium was exchanged with a PBS buffer (pH 7.5), with 0.5 mL of a high-speed centrifugal filtration column VIVA SPIN500 (manufactured by Sartorius), thereby giving an apolipoprotein A-II-containing preparation.

### Example 9

### Animal Experiment 1

Seven-week-old 22 male Balb/c mice (produced by Japan Charles River) were prepared as experimental animals. The apolipoprotein A-II prepared in Example 1 was administered intravenously once in a dose of 4 mg/kg to 8 mice (A-II administered group) out of the above mice. After 10 minutes, the 8 mice in the A-II administration group and other 8 mice (control group), that is, 16 mice in total, were administered once intraperitoneally with 700 mg/kg galactosamine hydrochloride (manufactured by Sigma) and 50 µg/kg lipopolysaccharide (E. coli 0111:B4, manufactured by Sigma) as fulminant hepatitis-inducing drugs. Six mice to which neither apolipoprotein A-II nor the fulminant hepatitis-inducing drugs were administered were used as the normal group.
Six hours after the apolipoprotein A-II preparation was administered, sera were separated by collecting bloods from abdominal aortas of the mice under anesthesia with carbon dioxide. The serum alanine aminotransferase (ALT) and aspartate aminotransferase (AST) levels that is, indicators of hepatic dysfunction in these sera, were measured to calculate mean ± standard error of the mean(SEM). Statistical analysis of ALT and AST levels was performed by Student's t-test (Excel, Microsoft) to examine the onset in the control group as compared with the normal group and the effect on the A-II administered group as compared with the control group.
The results are shown in Table 2.

**Table 2**

| | Group administered with (4 mg/kg) A-II in Example 1 | Control group | Normal group |
|---|---|---|---|
| ALT(IU/L) | 1053 ± 455* | 2896 ± 862## | 51 ± 4 |
| AST(IU/L) | 339 ± 99* | 698 ± 189## | 73 ± 5 |

| | | | |
|---|---|---|---|
| *: p<0.05 compared with the control group ##: p<0.01 compared with the normal group | | | |

As shown in Table 2, the serum ALT and AST levels in the control group increased significantly as compared with the normal group. However, the severe hepatic dysfunction recognized in the control group was suppressed significantly by the administration of A-II preparation. Through this experiment, the prophylactic effect of apolipoprotein A-II preparation on fulminant hepatitis was confirmed. A side effect considered attributable to administration of the A-II preparation was not observed in the A-II preparation administration group.

### Example 10

### Animal Experiment 2

Seven-week-old 18 female Balb/c mice (produced by Japan Charles River) were prepared as experimental animals. The apolipoprotein A-II prepared in Example 3 was administered intravenously once in a dose of 3 mg/kg to 8 mice (A-II administered group) out of the above mice. After 10 minutes, the 8 mice in the A-II administered group and other 6 mice (control group), that is, 14 mice in total, were administered once intravenously with 15 mg/kg concanavalin A type IV (manufactured by Sigma) as a fulminant hepatitis-inducing drug. Four mice to which neither the apolipoprotein A-II preparation nor concanavalin A was administered were used as the normal group.
Eight hours after the apolipoprotein A-II preparation was administered, sera were separated by collecting bloods from abdominal aortas of the mice under anesthesia with carbon dioxide. The serum alanine aminotransferase (ALT) and aspartate aminotransferase (AST) levels, which are indicators of hepatic dysfunction, were measured to calculate mean ± SEM. Statistical analysis of ALT and AST levels was performed by Student's t-test (Excel, Microsoft) to examine the onset in the control group as compared with the normal group and the effect on the A-II administered group as compared with the control group.
The results are shown in Table 3.

**Table 3**

| | Group administered with (3 mg/kg) A-II in Example 3 | Control group | Normal group |
|---|---|---|---|
| ALT(IU/L) | 785 ± 377* | 3388 ± 1156# | 33 ± 2 |
| AST(IU/L) | 668 ± 234* | 2450 ± 750# | 64 ± 2 |

| | | | |
|---|---|---|---|
| *: p<0.05 compared with the control group #: p<0.05 compared with the normal group | | | |

As shown in Table 3, the serum ALT and AST levels in the control group increased significantly as compared with the normal group.
However, the severe hepatic dysfunction recognized in the control group was suppressed significantly by the A-II preparation administration. In this experiment, the prophylactic effect of the apolipoprotein A-II preparation on fulminant hepatitis was showed. A side effect considered attributable to administration of the A-II preparation was not observed.

### Example 11

### Animal Experiment 3

Seven-week-old 18 female Balb/c mice (produced by Japan Charles River) were prepared as experimental animals. The apolipoprotein A-II prepared in Example 3 was administered intravenously once in a dose of 13 mg/kg to 8 mice (A-II administered group) out of the above mice. After 10 minutes, the 8 mice in the A-II administered group and other 6 mice, that is, 14 mice in total, were administered once intravenously with 15 mg/kg concanavalin A type IV (manufactured by Sigma) as a fulminant hepatitis-inducing drug. Four mice to which neither the apolipoprotein A-II preparation nor the fulminant hepatitis-inducing drug was administered were used as the normal group.
Eight hours after the apolipoprotein A-II preparation was administered, sera were separated by collecting bloods from abdominal aortas of the mice under anesthesia with carbon dioxide. The serum alanine aminotransferase (ALT) and aspartate aminotransferase (AST) levels, which are indicators of hepatic dysfunction, were measured to calculate mean ± SEM. Statistical analysis of ALT and AST levels was performed by Student's t-test (Excel, Microsoft) to examine the onset in the control group as compared with the normal group and the effect on the A-II administered group as compared with the control group.
The results are shown in Table 4.

**Table 4**

| | Group administered with (13 mg/kg) A-II in Example 3 | Control group | Normal group |
|---|---|---|---|
| ALT(IU/L) | 214 ± 33* | 3388 ± 1156# | 33 ± 2 |
| AST(IU/L) | 332 ± 28* | 2450 ± 750# | 64 ± 4 |

| | | | |
|---|---|---|---|
| *: p<0.05 compared with the control group #: p<0.05 compared with the normal group | | | |

As shown in Table 4, the serum ALT and AST levels in the control group increased significantly as compared with the normal group. However, the severe hepatic dysfunction recognized in the control group was suppressed significantly by the administration of A-II preparation. In this experiment, the prophylactic effect of the apolipoprotein A-II preparation on fulminant hepatitis was confirmed. A side effect considered attributable to administration of the A-II preparation was not observed.

### Example 12

### Animal Experiment 4

Eight-week-old 16 male Balb/c mice (produced by Japan Charles River) were prepared as experimental animals. The apolipoprotein A-II prepared in Example 1 was administered intravenously once in a dose of 300 mg/kg to 4 mice (A-II administered group) out of the above mice. After 10 minutes, the 4 mice in the A-II administered group and other 6 mice (control group), that is, 10 mice in total, were administered once intravenously with 200 µg/kg anti Fas antibody (manufactured by Becton Dickinson) as a fulminant hepatitis-inducing drug. Six mice to which neither the apolipoprotein A-II preparation nor the anti-Fas antibody was administered were used as the normal group.
Four hours after the apolipoprotein A-II preparation was administered, sera were separated by collecting bloods from abdominal aortas of the mice under anesthesia with carbon dioxide. The serum alanine aminotransferase (ALT) and aspartate aminotransferase (AST) levels, which are indicators of hepatic dysfunction, were measured to calculate mean ± SEM. Statistical analysis of ALT and AST levels was performed by Student's t-test (Excel, Microsoft) to examine the onset in the control group as compared with the normal group and the effect on the A-II administered group as compared with the control group.
The results are shown in Table 5.

**Table 5**

| | Group administered with (300 mg/kg) A-II in Example 1 | Control group | Normal group |
|---|---|---|---|
| ALT(IU/L) | 1433 ± 687** | 21199 ± 1156## | 58 ± 7 |
| AST(IU/L) | 769 ± 226** | 5567 ± 878## | 59 ± 3 |

| | | | |
|---|---|---|---|
| **: p<0.0 compared with the control group ##: p<0.01 compared with the normal group | | | |

As shown in Table 5, the serum ALT and AST levels in the control group increased significantly as compared with the normal group. However, the severe hepatic dysfunction recognized in the control group was suppressed significantly by the A-II preparation administration. In this experiment, the prophylactic effect of the apolipoprotein A-II preparation on fulminant hepatitis was confirmed. A side effect considered attributable to administration of the A-II preparation was not observed.

### Example 13

### Animal Experiment 5

Seven-week-old 32 female Balb/c mice (produced by Japan Charles River) were prepared as experimental animals.
Among them, 21 mice were administered once intravenously with 15 mg/kg concanavalin A type IV (manufactured by Sigma) respectively, and thereby prepared hepatic dysfunction-model mice which would develop fulminant hepatitis unless they were given some kind of treatment. One hour after concanavalin A was administered, the apolipoprotein A-II prepared in Example 1 was administered intravenously once in a dose of 100 mg/kg to the 10 hepatic dysfunction-model mice (A-II administered group). The 11 other hepatic dysfunction-model mice were not administered with the apolipoprotein A-II (control group). Eleven mice to which neither concanavalin A nor the apolipoprotein A-II was administered were used as the normal group.
Twenty-four hours after the apolipoprotein A-II preparation was administered, sera were separated by collecting bloods from abdominal aortas of the mice under anesthesia with carbon dioxide. The serum alanine aminotransferase (ALT) and aspartate aminotransferase (AST) levels, which are indicators of hepatic dysfunction, were measured to calculate mean ± SEM. Statistical analysis of ALT and AST levels was performed by Student's t-test (Excel, Microsoft) to examine the onset in the control group as compared with the normal group and the effect on the A-II administered group as compared with the control group.
The results are shown in Table 6.

**Table 6**

| | Group administered with (100 mg/kg) A-II in Example 1 | Control group | Normal group |
|---|---|---|---|
| ALT(IU/L) | 2387 ± 635* | 6582 ± 1598## | 33 ± 2 |
| AST(IU/L) | 1854 ± 406* | 4202 ± 968## | 58 ± 2 |

| | | | |
|---|---|---|---|
| *: p<0.05 compared with the control group ##: p<0.01 compared with the normal group | | | |

As shown in Table 6, the serum ALT and AST levels in the control group increased significantly as compared with the normal group. However, the severe hepatic dysfunction recognized in the control group was suppressed significantly by the A-II preparation administration. In this experiment, the therapeutic effect of the apolipoprotein A-II preparation on fulminant hepatitis was showed. A side effect considered attributable to administration of the A-II preparation was not observed.

### Example 14

### Animal Experiment 6

Seven-week-old 32 female Balb/c mice (produced by Japan Charles River) were prepared as experimental animals.
Among them, 21 mice were administered once intravenously with 15 mg/kg concanavalin A type IV (manufactured by Sigma) respectively, and thereby prepared hepatic dysfunction-model mice which would develop fulminant hepatitis unless they were given some kind of treatment. One hour after concanavalin A was administered, the apolipoprotein A-II prepared in Example 1 was administered intravenously once in a dose of 500 mg/kg to the 10 hepatic dysfunction-model mice (A-II administered group). The 11 other hepatic dysfunction-model mice were not administered with the apolipoprotein A-II (control group). Eleven mice to which neither concanavalin A nor the apolipoprotein A-II was administered were used as the normal group.
Twenty-four hours after the apolipoprotein A-II preparation was administered, sera were separated by collecting bloods from abdominal aortas of the mice under anesthesia with carbon dioxide. The serum alanine aminotransferase (ALT) and aspartate aminotransferase (AST) levels, which are indicators of hepatic dysfunction, were measured to calculate mean ± SEM. Statistical analysis of ALT and AST levels was performed by Student's t-test (Excel, Microsoft) to examine the onset in the control group as compared with the normal group and the effect on the A-II administered group as compared with the control group.
The results are shown in Table 7.

**Table 7**

| | Group administered with (500 mg/kg) A-II in Example 1 | Control group | Normal group |
|---|---|---|---|
| ALT(IU/L) | 1473 ± 865** | 6582 ± 1598## | 33 ± 2 |
| AST(IU/L) | 1257 ± 615* | 4202 ± 968## | 58 ± 2 |

| | | | |
|---|---|---|---|
| *: p<0.05 compared with the control group **: p<0.01 compared with the control group ##: p<0.01 compared with the normal group | | | |

As shown in Table 7, the serum ALT and AST levels in the control group increased significantly as compared with the normal group. However, the severe hepatic dysfunction recognized in the control group was suppressed significantly by the A-II preparation administration. In this experiment, the therapeutic effect of the apolipoprotein A-II preparation on fulminant hepatitis was confirmed. A side effect considered attributable to administration of the A-II preparation was not observed.

### Example 15

### Animal Experiment 7

Seven-week-old 24 male Balb/c mice (produced by Japan Charles River) were prepared as experimental animals.
Among them, 16 mice were administered once intravenously with 15 mg/kg concanavalin A type IV (manufactured by Sigma) respectively, and thereby prepared hepatic dysfunction-model mice which would develop acute hepatitis unless they were given some kind of treatment. One hour after concanavalin A was administered, the apolipoprotein A-II prepared in Example 1 was administered intravenously once in a dose of 500 mg/kg to the 8 hepatic dysfunction-model mice (A-II administered group). The 8 other hepatic dysfunction-model mice were not administered with the apolipoprotein A-II (control group). Eight mice to which neither concanavalin A nor the apolipoprotein A-II was administered were used as the normal group.
Twenty-four hours after the apolipoprotein A-II preparation was administered, sera were separated by collecting bloods from abdominal aortas of the mice under anesthesia with carbon dioxide. The serum alanine aminotransferase (ALT) and aspartate aminotransferase (AST) levels, which are indicators of hepatic dysfunction, were measured to calculate mean ± SEM. Statistical analysis of ALT and AST levels was performed by Student's t-test (Excel, Microsoft) to examine the onset in the control group as compared with the normal group and the effect on the A-II administered group as compared with the control group.
The results are shown in Table 8.

**Table 8**

| | Group administered with (500 mg/kg) A-II in Example 1 | Control group | Normal group |
|---|---|---|---|
| ALT(IU/L) | 1055 ± 281** | 4185 ± 736## | 55 ± 4 |
| AST(IU/L) | 936 ± 198** | 2831 ± 421## | 129 ± 13 |

| | | | |
|---|---|---|---|
| **: p<0.01 compared with the control group ##: p<0.01 compared with the normal group | | | |

As shown in Table 8, the serum ALT and AST levels in the control group increased significantly as compared with the normal group. However, the severe hepatic dysfunction recognized in the control group was suppressed significantly by the A-II preparation administration in Example 1. In this experiment, the therapeutic effect of the apolipoprotein A-II preparation on acute hepatitis was confirmed. A side effect considered attributable to administration of the A-II preparation was not observed.

### Industrial Applicability

In recent years, also in Japan, there are increasing cases where acute hepatitis proceeds to fulminant hepatitis. However, the progress into fulminant hepatitis can be prognosed in medical institutions from subjective symptoms of patients and careful diagnosis by medical doctors, and the therapeutic agent for acute hepatitis or the prophylactic/therapeutic agent for fulminant hepatitis in the present invention can be administered at an appropriate time, thereby treating acute hepatitis and preventing the hepatitis from becoming fulminant. Even if fulminant hepatitis is developed, its symptoms can be relieved by administering the pharmaceutical preparation of the present invention.

### Brief Description of Drawings

FIG. 1 shows SDS-PAGE electrophoretic profiles. In the figure, numerals on the left side indicate molecular weight (kDa). (1) shows molecular-weight markers; (2) the (unreduced) pharmaceutical preparation in Example 1; (3) the (unreduced) pharmaceutical preparation in Example 4; (4) the (reduced) pharmaceutical preparation in Example 1; (5) the (reduced) pharmaceutical preparation in Example 4; and (6) molecular-weight markers.
FIG. 2 shows western blotting electrophoretic profiles. (1) shows the (unreduced) pharmaceutical preparation in Example 1; (2) the (unreduced) pharmaceutical preparation in Example 4; (3) the (reduced) pharmaceutical preparation in Example 1; and (4) the (reduced) pharmaceutical preparation in Example 4.
FIG. 3 shows a peptide map of the pharmaceutical preparation (plasma-derived apolipoprotein A-II) in Example 1.
FIG. 4 shows a peptide map of the pharmaceutical preparation (recombinant apolipoprotein A-II) in Example 4. A peak (arrow) of His-Tag was detected in the vicinity of 17 minutes.

## Claims

1. A therapeutic agent for acute hepatitis or a prophylactic/therapeutic agent for fulminant hepatitis, which comprises apolipoprotein A-II as an active ingredient.

2. The therapeutic agent for acute hepatitis or prophylactic/therapeutic agent for fulminant hepatitis according to claim 1, which is a parenterally administered agent.

3. A therapeutic method for acute hepatitis or a prophylactic/therapeutic method for fulminant hepatitis, which comprises administering a composition containing apolipoprotein A-II as an active ingredient to a patient with acute hepatitis or fulminant hepatitis.
